# EUROPÄISCHE PATENTSCHRIFT

(11) **EP 3 313 482 B1**
(45) Veröffentlichungstag und Bekanntmachung des Hinweises auf die Patenterteilung: **24.06.2020**
(21) Anmeldenummer: 16722250.4
(22) Anmeldetag: 13.05.2016
(51) Int. Cl.: A61M 5/32

(54) **MEHRTEILIGE SICHERHEITSVORRICHTUNG FÜR EINE SPRITZE**
MULTI-PART SAFETY DEVICE FOR A SYRINGE
DISPOSITIF DE SÛRETÉ EN PLUSIEURS PARTIES POUR UNE SERINGUE

(30) Priorität: 26.06.2015 DE 102015110343
(43) Veröffentlichungstag der Anmeldung: 02.05.2018
(73) Patentinhaber: Gerresheimer Regensburg GmbH, 93047 Regensburg (DE)
(72) Erfinder: FRAAS, Andreas, 92224 Amberg (DE); VOGL, Maximilian, 92708 Mantel (DE)
(74) Vertreter: Hannke, Christian
(86) Internationale Anmeldenummer: PCT/EP2016/060863
(87) Internationale Veröffentlichungsnummer: WO 2016/206862

(56) Entgegenhaltungen:
- WO-A1-2013/134465
- DE-T5-112009 001 083
- DE-T5-112009 001 083
- US-A- 5 591 138
- US-A1- 2014 228 772
- US-B1- 6 238 371

## Beschreibung

Die Erfindung betrifft eine Sicherheitsvorrichtung zur Vermeidung von Stichverletzungen für eine Spritze mit einem Spritzenkörper und einem an dem distalen Ende des Spritzenkörpers angeordneten Stechmittel, umfassend ein sich entlang einer axialen Richtung (X) erstreckendes Hülsenelement, welches zumindest teilweise das Stechmittel und den Spritzenkörper umschließt, wobei das Hülsenelement drehbar an einem distalen Endbereich des Spritzenkörpers anordenbar ist.

Gattungsgemäße Sicherheitsvorrichtungen zur Vermeidung von Stichverletzungen sind aus dem Stand der Technik bekannt. Insbesondere bei vorgefüllten Spritzen ist die Verwendung derartiger Sicherheitsvorrichtungen sinnvoll. Die Handhabung derartiger Spritzen ist sehr einfach, da das Medium nicht vor der Anwendung in die Spritze transferiert werden muss. Weiterhin ist, selbst im Notfall, die Wahrscheinlichkeit der Anwendung eines falschen Medikaments sehr gering. Für Impfstoffe und zahlreiche andere Medikamente sind sie heutzutage das Primärpackmittel erster Wahl. Diese Spritzen sind üblicherweise aus Glas oder Kunststoff (beispielsweise COC, COP) hergestellt und müssen mit Schutzkappen ausgestattet sein, um Beschädigungen und/oder eine Kontaminierung der Kanüle vor der Anwendung der Spritze zu vermeiden. Darüber hinaus ist es wichtig, nach Gebrauch der Spritze die Kanüle zu sichern, um Stichverletzungen zu vermeiden. Dabei kann ein unvorsichtiges Wiederaufsetzen der Schutzkappe auf die Kanüle Stichverletzungen verursachen. Oft ist die entsprechende Schutzkappe nicht mehr auffindbar oder es wird vergessen, diese wiederaufzusetzen, wodurch ein vermeidbares Verletzungsrisiko gegeben ist. Eine Schutzvorrichtung mit integrierter Nadel ist aus US 2014 228 772 A1 bekannt. Weitere Schutzvorrichtungen sind aus US 5 591 138 A und US 6 238 371 B1 bekannt.

Demzufolge wurden Nadelschutzeinrichtungen entwickelt, welche fest mit der Spritze verbunden sind und die Nadel nach Verwendung der Spritze automatisch wieder aufnehmen. Eine solche Nadelschutzeinrichtung ist beispielsweise in DE 11 2009 001 083 T5 offenbart. Hierbei wird eine federangetriebene Sicherheitshülse gezeigt, welche in einem ausgefahrenen Zustand die Kanüle umgibt und diese gegen Verletzungen der Anwender sichert. Die Sicherheitshülse weist dabei eine Kurvenbahn auf, in welcher mindestens ein Führungsstift läuft, wodurch verschiedene Positionen der Sicherheitshülse in Abhängigkeit zur Nadelspitze realisiert werden können.

Der mindestens eine Führungsstift muss dabei über einen Kragen an der Frontgeometrie der Spritze befestigt werden oder muss auf andere Weise fest mit der Spritze verbunden werden. Der Kragen mit dem Führungsstift darf, um Manipulation oder Fehlbenutzung vorzubeugen, nicht oder nur schwer von der Spritze mit einer Kanüle entfernbar sein. Demnach ist ein entsprechend fester Sitz in axialer Richtung notwendig.

Daraus ergibt sich das Problem, dass die Sicherheitshülse bei einem fest sitzenden Führungsstift aufgrund der Kurvenbahn eine gewisse Rotation um die Längsachse des Spritzenkörpers erfährt. Die resultierende Rotation der auf der Haut des Patienten befindlichen Sicherheitshülse wird vom Patienten als unangenehm empfunden, da die Rotation der Sicherheitshülse die Haut um die Einstichstelle herum verdreht.

Zur Lösung dieses Problems wurden beispielsweise Sicherheitsvorrichtungen mit einer Führungskulisse und einem darin geführten Führungsstift entwickelt. Um nun eine Drehung des die Führungskulisse aufweisenden Hülsenelements zu vermeiden, wurde der Führungsstift rotierbar an dem Spritzenkörper befestigt. Eine solche Sicherheitsvorrichtung ist beispielsweise in der WO 2013/ 134465 A1 offenbart. Üblicherweise ist der Pin an einem Kragenelement angeordnet, welches rotierbar an dem distalen Ende des Spritzenkörpers angeordnet ist. In der Regel ist dies jedoch schwer ausführbar, da gerade Spritzen aus Glas in der Frontgeometrie erhebliche Toleranzschwankungen aufweisen. Ferner ist das entsprechende Kragenelement meist nur zur Anordnung an spezielle Frontgeometrien de Spritzenkörpers geeignet, wodurch der Anwendungsbereich der Sicherheitsvorrichtung eingeschränkt ist. Darüber hinaus ist ein rotierbarer Sitz des Pin-Elementes bei gleichzeitiger Sicherheit gegen Bewegung/Abziehen in Richtung der Längsrichtung des Spritzenkörpers herstellungstechnisch nur schwer realisierbar.

Aufgabe der vorliegenden Erfindung ist es demnach, eine Sicherheitsvorrichtung zur Vermeidung von Stichverletzungen für eine Spritze zur Verfügung zu stellen, welche die eingangs genannten Probleme löst.

Diese Aufgabe wird gelöst durch den Gegenstand des Anspruchs 1. Die Sicherheitsvorrichtung zeichnet sich dadurch aus, dass diese ein bei einer Anwendung der Spritze die Haut eines Patienten kontaktierendes Kontaktelement umfasst, welches an dem distalen Ende des Hülsenelements angeordnet ist und unabhängig von dem Hülsenelement rotierbar ist.

Vor der Anwendung der Spritze ist das Stechmittel in dem Hülsenelement angeordnet, wodurch das Stechmittel vor Beschädigungen und Kontamination geschützt wird. Ein Stechmittel kann in diesem Zusammenhang eine Kanüle, eine Nadel oder auch eine Lanzette sein. Bei einer Anwendung der Spritze tritt das Stechmittel aus dem Hülsenelement hervor. Dabei liegt das Kontaktelement an der Haut des Patienten an. Eine Rotation des Hülsenelements, um das Stechmittel hervortreten zu lassen, wird erfindungsgemäß nicht auf das Kontaktelement übertragen, da das Kontaktelement unabhängig von dem Hülsenelement rotierbar ist. Unter dem Begriff "unabhängig rotierbar" ist hierbei zu verstehen, dass die Haftreibung zwischen dem Hülsenelement und dem Kontaktelement kleiner ist als eine Haftreibung zwischen der Haut des Patienten und dem Kontaktelement. Das Hülsenelement kann somit rotieren, ohne dass diese Rotationsbewegung auf das Kontaktelement übertragen wird.

Vorzugsweise ist das Kontaktelement in einer Ebene senkrecht zu der axialen Richtung (X) rotierbar. Weiterhin bevorzugt ist das Kontaktelement in dieser Ebene an dem Hülsenelement angeordnet. In der Regel werden Spritzen derart angewendet, dass diese bei der Anwendung senkrecht zur Hautoberfläche ausgerichtet sind. Demnach ist es vorteilhaft, dass das Kontaktelement in einer Ebene senkrecht zu der axialen Richtung (X) rotiert, um eine Übertragung der Rotation des Hülsenelements auf die Haut des Patienten zu unterbinden.

Erfindungsgemäß umfasst die Sicherheitsvorrichtung ein Kragenelement, welches an dem distalen Endbereich des Spritzenkörpers anordenbar ist und die Sicherheitsvorrichtung an dem Spritzenkörper befestigt. Die Sicherheitsvorrichtung ist somit durch das Kragenelement fest an dem Spritzenkörper angeordnet. Ein derartiges Kragenelement kann einfach ausgestaltet werden, da dieses nicht rotierbar gegenüber dem Spritzenkörper ausgeführt werden muss. Insbesondere bei Spritzen aus Glas, welche herstellungsbedingt relativ große Toleranzen in der Spritzenfrontgeometrie aufweisen, kann das Kragenelement derart beschaffen sein, diese Toleranzen auszugleichen und somit eine optimale Befestigung der Sicherheitsvorrichtung zu gewährleisten. Ferner kann das Kragenelement derart ausgestaltet werden, dass eine Befestigung dessen an Spritzen mit unterschiedlicher Frontgeometrie ermöglicht ist. Insbesondere Kunststoffspritzen unterschiedlicher Hersteller können solche unterschiedlichen Frontgeometrien aufweisen. Vorstellbar wäre, das Kragenelement durch eine Klemmverbindung, eine Aufklipsverbindung, eine Klebeverbindung oder durch eine anderweitige Verbindung mit dem Spritzenkörper zu verbinden.

Vorzugsweise weist das Kragenelement zumindest einen Führungsvorsprung auf, welcher in zumindest einer Führungskulisse des Hülsenelements eingreift.

Es wäre aber auch denkbar, dass in dem distalen Endbereich des Spritzenkörpers zumindest ein Führungsvorsprung integral mit dem Spritzenkörper ausgebildet ist, wobei der zumindest eine Führungsvorsprung in zumindest einer Führungskulisse des Hülsenelements eingreift. Eine solche Ausführung ist insbesondere bei Kunststoffspritzen denkbar.

Erfindungsgemäß ist der Führungsvorsprung in der zumindest einen Führungskulisse des Hülsenelements bei einer Relativbewegung des Spritzenkörpers zu dem Hülsenelement im Wesentlichen entlang der axialen Richtung (X) geführt. Bei einer Anwendung der Spritze wird die Spritze mit der Sicherheitseinrichtung gegen die Haut des Patienten gedrückt. Durch die Relativbewegung des Spritzenkörpers zu dem Hülsenelement und die Führung des Führungsvorsprungs in der Führungskulisse wird eine Drehung des Hülsenelements entlang einer Umfangsrichtung (U) verursacht. Das Hülsenelement schiebt sich dadurch vorzugsweise über den Spritzenkörper, wodurch das Stechmittel durch eine entsprechende Öffnung in dem Hülsenelement hindurchtritt.

Vorteilhafterweise sind an dem Kragenelement und/oder an dem distalen Endbereich zwei sich diametral gegenüberliegende Führungsvorsprünge vorgesehen. Demzufolge würde auch das Hülsenelement zwei sich diametral gegenüberliegende Führungskulissen aufweisen, in denen jeweils ein Führungsvorsprung geführt wird. Dadurch würde eine besonders stabile Führung des Hülsenelements gewährleistet.

Erfindungsgemäß weist das Hülsenelement einen distalen Endabschnitt auf, welcher eine umlaufende Führungsnut aufweist. Erfindungsgemäß weist weiterhin das Kontaktelement einen Führungsvorsprung auf, welcher in die Führungsnut des Hülsenelements eingreift. Das Kontaktelement ist somit bezüglich der axialen Richtung (X) an dem Hülsenelement arretiert und weiterhin in einer Ebene senkrecht zu der axialen Richtung (X) rotierbar. Es wäre überdies denkbar, dass die Oberflächen der Führungsnut und/oder des Führungsvorsprungs eine Beschichtung aufweisen, welche die die Reibung zwischen den beiden verringert bzw. die Gleitfähigkeit erhöht. Dadurch würde eine leichtgängige Rotation des Kontaktelements erreicht.

Nach einer besonders bevorzugten Ausführungsform ist das Kontaktelement ringförmig ausgestaltet. Vorzugsweise liegen die Mittelsenkrechten des ringförmigen Kontaktelements und der Öffnung des Hülsenelements, durch welche das Stechmittel bei Anwendung der Spritze tritt, übereinander. Demzufolge tritt das Stechmittel bei Anwendung der Spritze durch den Mittelpunkt des ringförmigen Kontaktelements.

Gemäß einer weiteren bevorzugten Ausführungsform weist das Kontaktelement eine Stirnfläche auf, welche die Haut eines Patienten bei einer Anwendung der Spritze kontaktiert. Vorteilhafterweise weist diese Stirnfläche eine weiche und/oder glatte Haptik auf. Die Stirnfläche weist somit eine als angenehm für die Haut empfundene Haptik auf. Vorzugsweise kann das Kontaktelement aus einem solchen angenehm für die Haut empfundenen Material hergestellt werden. Es wäre auch denkbar, die Stirnfläche mit einer angenehm wirkenden Oberflächenstruktur auszustatten, beispielsweise durch Beschichtungen oder Ähnliches.

Gemäß einer bevorzugten Ausführungsform ist das Kragenelement im Wesentlichen als hohler Kreiszylinder ausgebildet. Bevorzugt weist der Kreiszylinder eine Mantelfläche auf, an der der zumindest eine Führungsvorsprung angeordnet ist. Vorzugsweise erstreckt sich der zumindest eine Führungsvorsprung radial von der Mantelfläche weg. Weiterhin bevorzugt ist der Führungsvorsprung als Kreiszylinder beziehungsweise als Stift ausgebildet.

Vorzugsweise ist der Spritzenkörper als hohler Kreiszylinder ausgestaltet und weist in seinem distalen Endbereich ein konisches Endstück auf, an welchem das Stechmittel angeordnet ist. Bevorzugt ist an dem konischen Endstück ein Vorsprung ausgebildet, an welchem eine Stirnfläche des distalen Endes des Kragenelements eingreifbar ist, wodurch das Kragenelement und somit die Sicherheitsvorrichtung in axialer Richtung arretierbar ist. Weiterhin bevorzugt ist auch die Sicherheitsvorrichtung im Wesentlichen in Form eines hohlen Kreiszylinders ausgestaltet.

Vorzugsweise weist die Sicherheitsvorrichtung mindestens ein Federelement auf, das mit dem Spritzenkörper wirkverbunden ist und der Relativbewegung des Spritzenkörpers zu dem Hülsenelement bzw. der Sicherheitsvorrichtung entgegenwirkt. Demnach bleibt das Stechmittel bis zur vorgesehenen Anwendung innerhalb des Hülsenelements. Bei der Anwendung muss das Hülsenelement gegen die Federkraft verschoben werden, damit die Kanüle durch die Öffnung des Hülsenelements hindurchtreten kann. Durch die Führung des Führungsvorsprungs in der Führungskulisse rotiert das Hülsenelement entlang der Umfangsrichtung (U). Nach Gebrauch der Spritze schiebt sich automatisch, angetrieben durch die Federkraft des Federelements, das Hülsenelement wieder über das Stechmittel, wobei das Hülsenelement durch die Führung des Führungsvorsprungs in der Führungskulisse nun entgegen der Umfangsrichtung (U) rotiert. Der Anwender ist somit vor Stichverletzungen mit der gebrauchten kontaminierten Kanüle geschützt. Bevorzugt umfasst das Federelement eine Spiralfeder. Denkbar sind aber auch anderweitige Federarten, wie beispielsweise Schenkelfedern oder Torsionsfedern. Ferner wäre vorstellbar, das Federelement als ein Elastomer auszubilden.

Nach einem weiteren vorteilhaften Gedanken der Erfindung umfasst die zumindest eine Führungskulisse einen ersten und einen zweiten Kulissenbereich, die durch eine entlang der axialen Richtung (X) des Spritzenkörpers verlaufende fiktive Trennlinie voneinander getrennt sind, wobei der Führungsvorsprung in einer Ausgangsstellung in dem ersten Kulissenbereich anordenbar ist und von dem ersten in den zweiten Kulissenbereich in eine Endstellung durch Überschreiten der Trennlinie überführbar ist, wenn ein distales Ende des Stechelements bei der Relativbewegung von Spritzenkörper zu Hülsenelement auf der Höhe der distalen Öffnung des Hülsenelements angeordnet ist.

Demnach ist der Führungsvorsprung von dem ersten Kulissenbereich in den zweiten Kulissenbereich überführbar. Diese Überführung findet statt, wenn der Führungsvorsprung eine fiktive Trennlinie, welche den ersten und den zweiten Kulissenbereich voneinander trennt, überschreitet. Befindet sich der Führungsvorsprung in dem ersten Kulissenbereich, also in einer Ausgangsstellung, so wurde die Spritze noch nicht ausgelöst, d.h. das Stechmittel hat die Sicherheitsvorrichtung noch nicht verlassen. Befindet sich der Führungsvorsprung in dem zweiten Kulissenbereich, so ist das Stechmittel aus der Sicherheitsvorrichtung bereits ausgetreten, so dass eine Injektion möglich ist. Beim Übergang von dem ersten Kulissenbereich zu dem zweiten Kulissenbereich, also genau dann, wenn der Führungsvorsprung die Trennlinie überschreitet, befindet sich das distale Ende des Stechmittels auf der Höhe der distalen Öffnung der Sicherheitsvorrichtung.

Bevorzugt ist der Führungsvorsprung von dem zweiten Kulissenbereich mittels einer Kulisse des zweiten Kulissenbereichs in einen Endbereich überführbar, in welchem eine Relativbewegung des Hülsenelements zu dem Spritzenkörper im Wesentlichen entlang der axialen Richtung (X) zumindest eingeschränkt ist. Durch eine derartige Ausgestaltung ist ein weiteres Verschieben des Hülsenelements relativ zum Spritzenkörper zumindest eingeschränkt, bevorzugt verhindert. Demzufolge ist ein weiteres Austreten des Stechmittels aus der Sicherheitsvorrichtung nach der Anwendung der Spritze unterbunden.

Weitere Vorteile, Ziele und Eigenschaften der vorliegenden Erfindung werden anhand nachfolgender Beschreibung der anliegenden Figuren erläutert. Gleichartige Komponenten können in den verschiedenen Ausführungsformen gleiche Bezugszeichen aufweisen.

In den Figuren zeigen:
- Fig.1: eine isometrische Ansicht einer Spritze mit Sicherheitsvorrichtung;
- Fig.2: eine Schnittdarstellung einer Spritze mit Sicherheitsvorrichtung;
- Fig.3: eine Schnittdarstellung einer Spritze mit Sicherheitsvorrichtung;
- Fig.4: eine Seitenansicht einer Sicherheitsvorrichtung;
- Fig.5: eine Schnittdarstellung einer Sicherheitsvorrichtung.

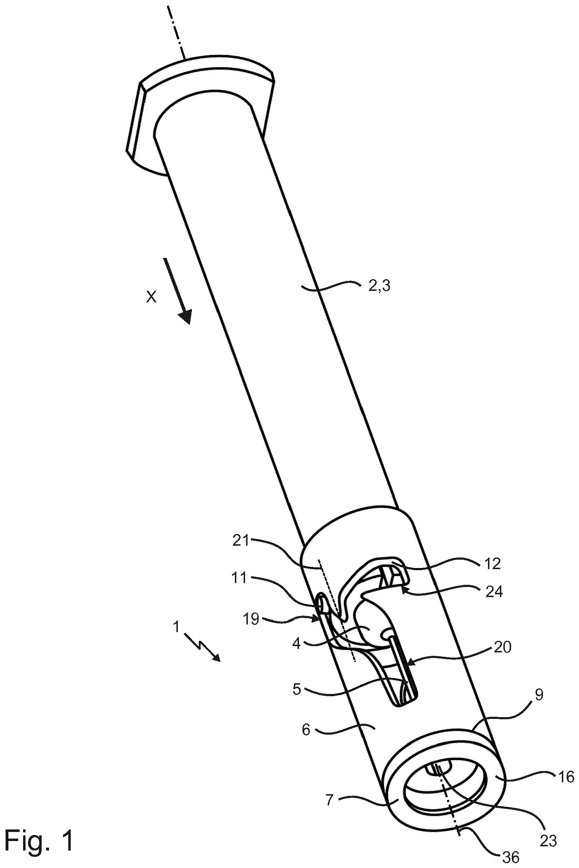

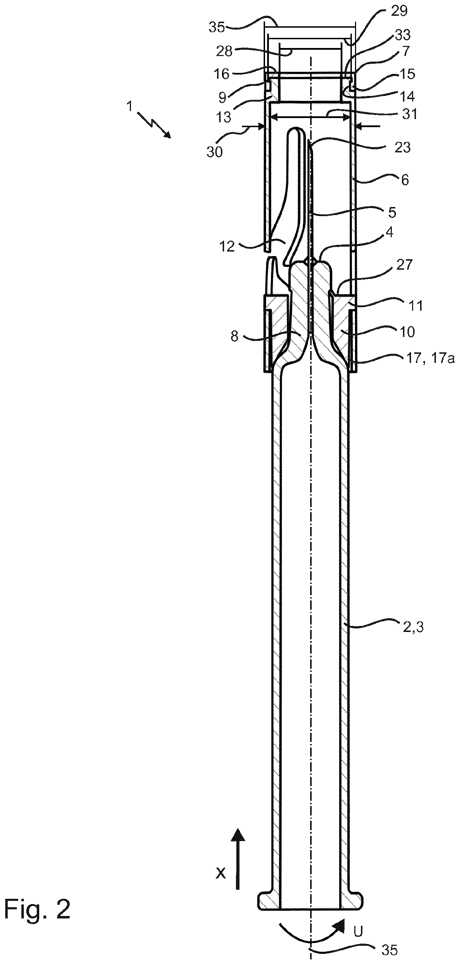

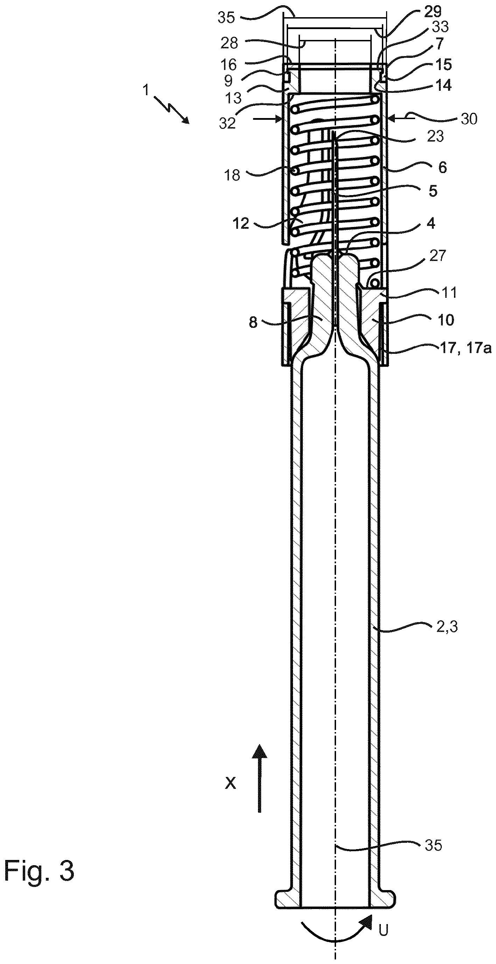

In Fig. 1 ist eine isometrische Ansicht einer Spritze (2) mit einer Sicherheitsvorrichtung (1) zur Vermeidung von Stichverletzungen gezeigt. In den Figuren 2 und 3 ist jeweils eine Schnittdarstellung einer Spritze (2) mit einer Sicherheitsvorrichtung (1) gezeigt.

Die Spritze (2) umfasst einen Spritzenkörper (3), welcher als hohler Kreiszylinder ausgestaltet ist. Der Spritzenkörper weist einen distalen Endbereich (8) mit einem distalen Ende (4) auf. An dem distalen Ende (4) ist ein Stechmittel (5) angeordnet. Dieses Stechmittel (5) ist über eine Bohrung in dem distalen Endbereich (8) mit dem Hohlraum des Spritzenkörpers (3) verbunden, so dass das zu injizierende Medium bei einer Anwendung der Spritze (2) aus dem Hohlraum durch das Stechmittel (5) treten kann. Der distale Endbereich (8) ist als konisches Endstück ausgestaltet, welches einen kleineren Außendurchmesser als der Spritzenkörper (3) aufweist. Weiterhin weist die Spritze einen Übergangsbereich (25) auf, in dem der Außendurchmesser des Spritzenkörpers (3) in den Außendurchmesser des Endstücks übergeht.

Die Sicherheitsvorrichtung (1) zur Vermeidung von Stichverletzungen für eine Spritze (2) mit einem Spritzenkörper (3) und einem an dem distalen Ende (4) des Spritzenkörpers (3) angeordneten Stechmittel (5), umfasst ein sich entlang einer axialen Richtung (X) erstreckendes Hülsenelement (6), welches zumindest teilweise das Stechmittel (5) und den Spritzenkörper (3) umschließt, wobei das Hülsenelement (6) drehbar an einem distalen Endbereich (8) des Spritzenkörpers (3) anordenbar ist. Die Sicherheitsvorrichtung (1) umfasst ein bei einer Anwendung der Spritze (2) die Haut eines Patienten kontaktierendes Kontaktelement (7), welches an dem distalen Ende (9) des Hülsenelements (6) angeordnet ist und unabhängig von dem Hülsenelement (6) rotierbar ist, wobei das Kontaktelement (7) in einer Ebene senkrecht zu der axialen Richtung (X) rotierbar ist.

Die Sicherheitsvorrichtung (1) umfasst weiterhin ein Kragenelement (10), welches an dem distalen Endbereich (8) des Spritzenkörpers (3) anordenbar ist. Die Sicherheitsvorrichtung (1) wird durch das Kragenelement (10) an dem Spritzenkörper (3) befestigt. Das Kragenelement (10) ist weiter im Wesentlichen als hohler Kreiszylinder (17) ausgebildet. Die Arretierung in axialer Richtung wird durch einen Vorsprung (26) bzw. eine Verdickung an dem distalen Ende (4) des Spritzenkörpers (3) ermöglicht, an welchem das Kragenelement (10) mit seinem distalen Ende (27) anliegt. Ferner weist der Kreiszylinder (17) eine Mantelfläche (17a) auf, an der zwei Führungsvorsprünge (11) angeordnet sind. Die Führungsvorsprünge (11) erstrecken sich radial von der Mantelfläche (17a) nach außen weg und sind diametral gegenüberliegend angeordnet. Weiterhin sind diese als Kreiszylinder beziehungsweise als Stift ausgebildet. Diese beiden Führungsvorsprünge (11) greifen in jeweils eine Führungskulisse (12) des Hülsenelements (6) ein und sind in diesen bei einer Relativbewegung des Spritzenkörpers (3) zu dem Hülsenelement (6) im Wesentlichen entlang der axialen Richtung (X) geführt.

In Fig. 3 ist weiterhin eine Sicherheitsvorrichtung (1) dargestellt, welche ein Federelement (18) in Form einer Spiralfeder aufweist, das mit dem Spritzenkörper (3) wirkverbunden ist und der Relativbewegung des Hülsenelements (6) zu der Sicherheitsvorrichtung (1) entgegenwirkt.

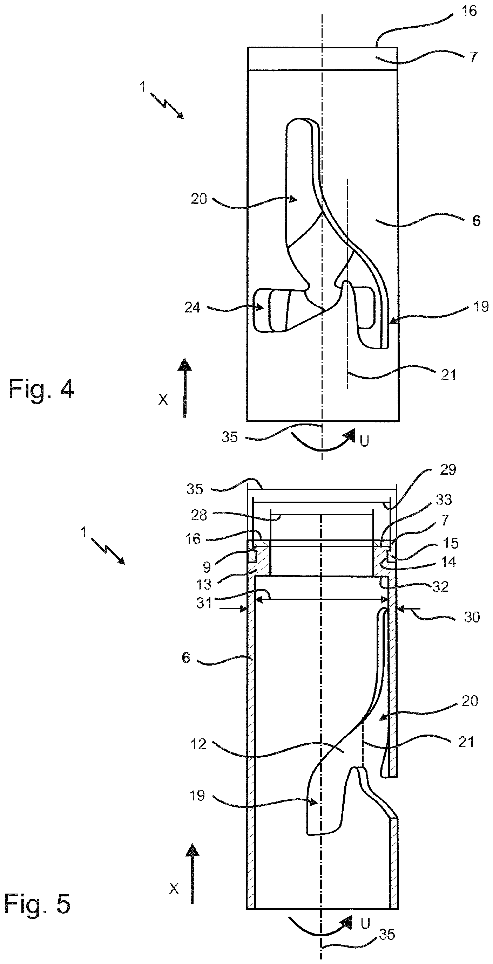

Das Hülsenelement (6) und das Kontaktelement (7) sind in den Figuren 4 und 5 detailliert dargestellt, wobei Fig. 4 eine Seitenansicht und Fig. 5 eine Schnittdarstellung des Hülsenelements (6) und des Kontaktelement (7) zeigen. Das Hülsenelement (6) ist im Wesentlichen zylindrisch ausgebildet und umfasst einen distalen Endabschnitt (13), welcher einen Innendurchmesser (28) und einen Außendurchmesser (29) aufweist. Sowohl der Innendurchmesser (28) als auch der Außendurchmesser (29) des distalen Endabschnitts (13) sind kleiner als ein Innendurchmesser (31) und ein Außendurchmesser (30) des anschließenden Abschnitts des Hülsenelements (6). Ferner weist das Hülsenelement (6) in diesem distalen Endabschnitt (13) eine größere Wandstärke auf. Dadurch wird zum einen eine Auflagefläche (32) ausgebildet, an welcher das Federelement (18) in axialer Richtung (X) anliegt. Zum anderen ist eine Führungsnut (14) in der Mantelfläche des distalen Endabschnitts (13) ausgebildet.

Das Kontaktelement (10) ist ringförmig ausgestaltet und weist eine Stirnfläche (16) auf, welche die Haut eines Patienten bei einer Anwendung der Spritze (2) kontaktiert. Diese Stirnfläche (16) kann eine weiche und/oder glatte Haptik aufweisen, wodurch diese angenehm für die Haut empfunden wird. Die der Stirnfläche (16) gegenüberliegende Fläche des Kontaktelements (10) liegt auf einer Stirnfläche (33) des distalen Endabschnitts (13) an. Ausgehend von dieser gegenüberliegenden Fläche erstreckt sich in axialer Richtung (X) ein Führungsfortsatz (34), an welchem ein Führungsvorsprung (15) angeordnet ist. Dieser Führungsvorsprung (15) greift in die umlaufende Führungsnut (14) des Hülsenelements ein. Der Führungsfortsatz (34) ist derart ausgebildet, dass der Außendurchmesser (35) des Kontaktelements (7) dem Außendurchmesser (30) des Hülsenelements (6) entspricht. Ferner liegen die Mittelachsen des ringförmigen Kontaktelements (7) und die distale Öffnung (23) des Hülsenelements (6) übereinander, so dass das Stechmittel bei einer Anwendung der Spritze durch den Mittelpunt des ringförmigen Kontaktelements (7) tritt.

Das Stechmittel (5) bleibt bis zur vorgesehenen Anwendung der Spritze (2) innerhalb des Hülsenelements (6). Bei einer Anwendung wird die Spritze an die Haut des Patienten gepresst. Das Hülsenelement (6) muss gegen die Federkraft des Federelements (18) verschoben werden, damit das Stechmittel (5) durch die distale Öffnung (23) des Hülsenelements (6) und durch das ringförmige Kontaktelement (7) hindurchtreten kann. Das Hülsenelement wird dabei über den distalen Endbereich (8) des Spritzenkörpers (3) geschoben. Durch die Führung des Führungsvorsprungs (11) in der Führungskulisse (12) rotiert das Hülsenelement (6) entlang der Umfangsrichtung (U). Durch den Anpressdruck ist die Haftreibung zwischen der Haut des Patienten und dem Kontaktelement (7) größer als die Haftreibung zwischen dem Kontaktelement (7) und dem Hülsenelement (6). Demnach wird dir Rotation des Hülsenelements (6) nicht auf das Kontaktelement (7) beziehungsweise die Haut des Patienten übertragen. Nach Gebrauch der Spritze (2) schiebt sich automatisch, angetrieben durch die Federkraft des Federelements (22), das Hülsenelement (6) wieder über das Stechmittel (5). Durch die Führung der Führungsvorsprünge (9) in den Führungskulissen (10) rotiert das Hülsenelement (6) in entgegen der Umfangsrichtung (U), wobei diese Rotation aus oben genannten Gründen ebenso wenig auf die Haut des Patienten übertragen wird. Bei einer solchen Drehung entlang oder gegen die Umfangsrichtung (U) entspricht die Drehachse der Mittelachse (36) der Sicherheitsvorrichtung.

Die Führungskulissen (12) des Hülsenelements (6) umfassen in der gezeigten Ausführungsform einen ersten (19) und einen zweiten Kulissenbereich (20), welche durch eine entlang des axialen Richtung (X) des Spritzenkörpers (3) verlaufende fiktive Trennlinie (21) voneinander getrennt sind, wobei der Führungsvorsprung (11) in einer Ausgangsstellung in dem ersten Kulissenbereich (19) anordenbar ist und von dem ersten (19) in den zweiten Kulissenbereich (20) in eine Endstellung durch Überschreiten der Trennlinie (25) überführbar ist, wenn ein distales Ende (22) des Stechmittels (5) bei der Relativbewegung von Spritzenkörper (3) zu Hülsenelement (6) auf der Höhe der distalen Öffnung (23) des Hülsenelements (6) angeordnet ist.

Ferner weist das Hülsenelement (6) einen Endbereich (24) auf. Die Führungsvorsprünge (11) sind dabei von dem zweiten Kulissenbereich (20) mittels einer Kulisse des zweiten Kulissenbereichs (20) in einen Endbereich (24) überführbar. In diesem Endbereich (24) ist eine Relativbewegung des Hülsenelements (6) zu dem Spritzenkörper (3) im Wesentlichen entlang der axialen Richtung (X) zumindest eingeschränkt.

### Bezugszeichenliste

- 1: Sicherheitsvorrichtung
- 2: Spritze
- 3: Spritzenkörper
- 4: distales Ende des Spritzenkörpers
- 5: Stechmittel
- 6: Hülsenelement
- 7: Kontaktelement
- 8: distaler Endbereich des Spritzenkörpers
- 9: distales Ende des Hülsenelements
- 10: Kragenelement
- 11: Führungsvorsprung
- 12: Führungskulisse
- 13: distaler Endabschnitt des Hülsenelements
- 14: Führungsnut
- 15: Führungsvorsprung
- 16: Stirnfläche des Kontaktelements
- 17: Kreiszylinder
- 17a: Mantelfläche des Kreiszylinders
- 18: Federelement
- 19: erster Kulissenbereich
- 20: zweiter Kulissenbereich
- 21: Trennlinie
- 22: distales Ende des Stechmittels
- 23: distalen Öffnung des Hülsenelements
- 24: Endbereich
- 25: Übergangsbereich
- 26: Vorsprung
- 27: distales Ende des Kragenelements
- 28: Innendurchmesser des distalen Endabschnitts
- 29: Außendurchmesser des distalen Endabschnitts
- 30: Außendurchmesser des Hülsenelements
- 31: Innendurchmesser des Hülsenelements
- 32: Auflagefläche
- 33: Stirnfläche des distalen Endabschnitts
- 34: Führungsfortsatz
- 35: Außendurchmesser des Kontaktelements
- 36: Mittelachse der Sicherheitsvorrichtung
- X: axiale Richtung
- U: Umfangsrichtung
- R: radiale Richtung

## Patentansprüche

1. Spritzenkörper (3) einer Spritze (2) mit einem an dessen distalen Ende (4) angeordneten Stechmittel (5), wobei an dem Spritzenkörper (3) eine Sicherheitsvorrichtung (1) zur Vermeidung von Stichverletzungen angeordnet ist, die ein sich entlang einer axialen Richtung (X) erstreckendes Hülsenelement (6) umfasst, welches zumindest teilweise das Stechmittel (5) und den Spritzenkörper (3) umschließt, wobei das Hülsenelement (6) einen distalen Endabschnitt (13) und einen daran anschließenden weiteren Abschnitt aufweist und an einem distalen Endbereich (8) des Spritzenkörpers (3) angeordnet ist,
**dadurch gekennzeichnet, dass**
die Sicherheitsvorrichtung (1) ein Kragenelement (10) umfasst, durch welches die Sicherheitsvorrichtung (1) an dem distalen Endbereich (8) des Spritzenkörpers (3) fest angeordnet ist, wobei das Hülsenelement (6) zumindest eine Führungskulisse (12) umfasst, wobei bei einer Relativbewegung des Spritzenkörpers (3) zu dem Hülsenelement (6) im Wesentlichen entlang der axialen Richtung (X) durch eine Führung zumindest eines Führungsvorsprungs (11) in der zumindest einen Führungskulisse (12) das Hülsenelement (6) relativ zu dem Spritzenkörper (3) entlang der Umfangsrichtung (U) rotiert, wobei die Sicherheitsvorrichtung (1) ein bei einer Anwendung der Spritze (2) die Haut eines Patienten kontaktierendes Kontaktelement (7) umfasst, welches an dem distalen Ende (9) des Hülsenelements (6) angeordnet ist und unabhängig von dem Hülsenelement (6) entlang der Umfangsrichtung (U) rotierbar ist, wodurch eine Rotation des Hülsenelements (6) während der Anwendung der Spritze (2) nicht auf das Kontaktelement (7) übertragbar ist, wobei eine umlaufende Führungsnut (14) in einer Mantelfläche des distalen Endabschnitts (13) ausgebildet ist, und wobei das Kontaktelement (7) einen Führungsvorsprung (15) aufweist, welcher in die Führungsnut (14) des Hülsenelements (6) eingreift, wobei eine Wandstärke des Hülsenelements (6) in dem distalen Endabschnitt (13) größer ist als in dem weiteren Abschnitt.

2. Spritzenkörper (3) nach Anspruch 1,
**dadurch gekennzeichnet, dass**
das Kontaktelement (7) in einer Ebene senkrecht zu der axialen Richtung (X) rotierbar ist.

3. Spritzenkörper (3) nach Anspruch 1,
**dadurch gekennzeichnet, dass**
das Kragenelement (10) zumindest einen Führungsvorsprung (11) aufweist, welcher in der zumindest einen Führungskulisse (12) des Hülsenelements (6) eingreift.

4. Spritzenkörper (3) nach einem der Ansprüche 1-2,
**dadurch gekennzeichnet, dass**
in dem distalen Endbereich (8) des Spritzenkörpers (3) zumindest ein Führungsvorsprung (11) integral mit dem Spritzenkörper (3) ausgebildet ist, wobei der zumindest eine Führungsvorsprung (11) in die zumindest eine Führungskulisse (12) des Hülsenelements (6) eingreift.

5. Spritzenkörper (3) nach einem der vorhergehenden Ansprüche,
**dadurch gekennzeichnet, dass**
das Kontaktelement (7) ringförmig ausgestaltet ist.

6. Spritzenkörper (3) nach einem der vorhergehenden Ansprüche,
**dadurch gekennzeichnet, dass**
das Kontaktelement (7) eine Stirnfläche (16) aufweist, welche die Haut eines Patienten bei einer Anwendung der Spritze (2) kontaktiert, wobei die Stirnfläche (16) eine weiche und/oder glatte Haptik aufweist.

7. Spritzenkörper (3) nach einem der vorhergehenden Ansprüche,
**dadurch gekennzeichnet, dass**
das Kragenelement (10) im Wesentlichen als hohler Kreiszylinder (17) ausgebildet ist, wobei der Kreiszylinder (17) eine Mantelfläche (17a) aufweist, an der der zumindest eine Führungsvorsprung (11) angeordnet ist.

8. Spritzenkörper (3) nach einem der vorhergehenden Ansprüche,
**dadurch gekennzeichnet, dass**
die Sicherheitsvorrichtung (1) mindestens ein Federelement (18) aufweist, das mit dem Spritzenkörper (3) wirkverbunden ist und der Relativbewegung des Spritzenkörpers (3) zu dem Hülsenelement (6) entgegenwirkt.

9. Spritzenkörper (3) nacheinem der vorhergehenden Ansprüche,
**dadurch gekennzeichnet, dass**
die Führungskulisse (12) einen ersten (19) und einen zweiten Kulissenbereich (20) umfasst, die durch eine entlang des axialen Richtung (X) des Spritzenkörpers (3) verlaufende fiktive Trennlinie (21) voneinander getrennt sind, wobei der Führungsvorsprung (11) in einer Ausgangsstellung in dem ersten Kulissenbereich (19) anordenbar ist und von dem ersten (19) in den zweiten Kulissenbereich (20) in eine Endstellung durch Überschreiten der Trennlinie (21) überführbar ist, wenn ein distales Ende (22) des Stechmittels (5) bei der Relativbewegung von Spritzenkörper (3) zu dem Hülsenelement (6) auf der Höhe der distalen Öffnung (23) des Hülsenelements (6) angeordnet ist.

10. Spritzenkörper (3) nach Anspruch 9,
**dadurch gekennzeichnet, dass**
der zumindest eine Führungsvorsprung (11) von dem zweiten Kulissenbereich (20) mittels einer Kulisse des zweiten Kulissenbereichs (20) in einen Endbereich (24) überführbar ist, in welchem eine Relativbewegung des Hülsenelements (6) zu dem Spritzenkörper (3) im Wesentlichen entlang der axialen Richtung (X) zumindest eingeschränkt ist.

## Claims

1. Syringe body (3) of a syringe (2) having a piercing means (5) arranged at its distal end (4), wherein a safety device (1), for avoiding stab wounds, is arranged on the syringe body (3), comprising a sleeve element (6) which extends along an axial direction (X) and at least partially encloses the piercing means (5) and the syringe body (3), the sleeve element (6) comprising a distal end portion (13) and an adjacent another portion and is arranged on a distal end region (8) of the syringe body (3),
**characterised in that**
the safety device (1) comprises a collar element (10), by means of which the safety device (1) is rigidly arranged on the distal end region (8) of the syringe body (3), wherein the sleeve element (6) comprises a guide track (12), wherein during a movement of the syringe body (3) relative to the sleeve element (6) substantially along the axial direction (X), the guidance of at least one guide projection (11) in the at least one guide track (12) causes the sleeve element (6) to rotate along the circumferential direction (U), the safety device (1) comprising a contact element (7) which contacts a patient's skin when the syringe (2) is used and which is arranged at the distal end (9) of the sleeve element (6) and is rotatable independently of the sleeve element (6) along the circumferential direction (U), whereby a rotation of the sleeve element (6) is not transferred to the contact element (7) during the use of the syringe (2), a circumferential guide groove (14) being formed in a lateral surface of the distal end portion (13), and the contact element (7) comprising a guide projection (15) which engages in the guide groove (14) of the sleeve element (6), wherein the wall thickness of the sleeve element (6) in the distal end portion (13) is greater than in the another portion.

2. Syringe body (3) according to claim 1,
**characterised in that**
the contact element (7) is rotatable in a plane perpendicular to the axial direction (X).

3. Syringe body (3) according to claim 1,
**characterised in that**
the collar element (10) comprises at least one guide projection (11), which engages in the at least one guide track (12) of the sleeve element (6).

4. Syringe body (3) according to either claim 1 or claim 2,
**characterised in that**
at least one guide projection (11) is formed integrally with the syringe body (3) in the distal end region (8) of the syringe body (3), the at least one guide projection (11) engaging in the at least one guide track (12) of the sleeve element (6).

5. Syringe body (3) according to any of the preceding claims,
**characterised in that**
the contact element (7) is annular.

6. Syringe body (3) according to any of the preceding claims,
**characterised in that**
the contact element (7) comprises a front face (16) that contacts a patient's skin when the syringe (2) is used, the front face (16) having a soft and/or smooth feel.

7. Syringe body (3) according to any of the preceding claims,
**characterised in that**
the collar element (10) is substantially formed as a hollow circular cylinder (17), the circular cylinder (17) comprising a lateral surface (17a), on which the at least one guide projection (11) is arranged.

8. Syringe body (3) according to any of the preceding claims,
**characterised in that**
the safety device (1) comprises at least one spring element (18), which is operatively connected to the syringe body (3) and counteracts the movement of the syringe body (3) relative to the sleeve element (6).

9. Syringe body (3) according to any of the preceding claims,
**characterised in that**
the guide track (12) comprises a first (19) and a second track region (20), which are separated from one another by a fictive separating line (21) extending along the axial direction (X) of the syringe body (3), it being possible for the guide projection (11) to be arranged in a starting position in the first track region (19) and to be moved from the first track region (19) into an end position in the second track region (20) by passing the separating line (21) when a distal end (22) of the piercing means (5) is arranged at the level of the distal opening (23) of the sleeve element (6) as the syringe body (3) is moved relative to the sleeve element (6).

10. Syringe body (3) according to claim 9,
**characterised in that**
the at least one guide projection (11) can be moved, by means of a track of the second track region (20), from the second track region (20) into an end region (24) in which a movement of the sleeve element (6) relative to the syringe body (3) is at least limited, substantially along the axial direction (X).

## Revendications

1. Corps de seringue (3) d'une seringue (2) comportant un moyen piquant (5) disposé sur son extrémité distale (4), dans lequel, sur le corps de seringue (3), est disposé un dispositif de sécurité (1) destiné à éviter les blessures par piqûre, lequel dispositif de sécurité (1) comporte un élément manchon (6) s'étendant le long d'une direction axiale (X), lequel entoure au moins partiellement le moyen piquant (5) et le corps de seringue (3), dans lequel l'élément manchon (6) présente une section d'extrémité distale (13) et une autre section s'y raccordant, et est disposé sur une région d'extrémité distale (8) du corps de seringue (3),
**caractérisé par le fait que**
le dispositif de sécurité (1) comporte un élément collier (10) au moyen duquel le dispositif de sécurité (1) est disposé de manière fixe sur la région d'extrémité distale (8) du corps de seringue (3), dans lequel l'élément manchon (6) comporte au moins une coulisse de guidage (12), dans lequel, lors d'un déplacement relatif du corps de seringue (3) par rapport à l'élément manchon (6) sensiblement le long de la direction axiale (X), par un guidage d'au moins une saillie de guidage (11) dans ladite au moins une coulisse de guidage (12), l'élément manchon (6) tourne le long de la direction périphérique (U) par rapport au corps de seringue (3), dans lequel le dispositif de sécurité (1) comporte un élément de contact (7) venant en contact avec la peau d'un patient lors d'une utilisation de la seringue (2), lequel élément de contact (7) est disposé sur l'extrémité distale (9) de l'élément manchon (6) et est apte à tourner le long de la direction périphérique (U) indépendamment de l'élément manchon (6), ce par quoi une rotation de l'élément manchon (6) durant l'utilisation de la seringue (2) n'est pas transférable à l'élément de contact (7), dans lequel une rainure de guidage périphérique (14) est formée dans une surface d'enveloppe de la section d'extrémité distale (13), et dans lequel l'élément de contact (7) présente une saillie de guidage (15), laquelle s'engage dans la rainure de guidage (14) de l'élément manchon (6), dans lequel une épaisseur de paroi de l'élément manchon (6) est plus grande dans la section d'extrémité distale (13) que dans l'autre section.

2. Corps de seringue (3) selon la revendication 1,
**caractérisé par le fait que**
l'élément de contact (7) est apte à tourner dans un plan perpendiculaire à la direction axiale (X).

3. Corps de seringue (3) selon la revendication 1,
**caractérisé par le fait que**
l'élément collier (10) présente au moins une saillie de guidage (11), laquelle s'engage dans ladite au moins une coulisse de guidage (12) de l'élément manchon (6).

4. Corps de seringue (3) selon l'une des revendications 1 et 2,
**caractérisé par le fait que**
dans la région d'extrémité distale (8) du corps de seringue (3), au moins une saillie de guidage (11) est formée d'un seul tenant avec le corps de seringue (3), dans lequel ladite au moins une saillie de guidage (11) s'engage dans ladite au moins une coulisse de guidage (12) de l'élément manchon (6).

5. Corps de seringue (3) selon l'une des revendications précédentes,
**caractérisé par le fait que**
l'élément de contact (7) est configuré de forme annulaire.

6. Corps de seringue (3) selon l'une des revendications précédentes,
**caractérisé par le fait que**
l'élément de contact (7) présente une face frontale (16), laquelle vient en contact avec la peau d'un patient lors de l'utilisation de la seringue (2), la face frontale (16) présentant une haptique souple et/ou lisse.

7. Corps de seringue (3) selon l'une des revendications précédentes,
**caractérisé par le fait que**
l'élément collier (10) est formé sensiblement en tant que cylindre creux à base circulaire (17), le cylindre à base circulaire (17) présentant une surface d'enveloppe (17a) sur laquelle est disposée ladite au moins une saillie de guidage (11).

8. Corps de seringue (3) selon l'une des revendications précédentes,
**caractérisé par le fait que**
le dispositif de sécurité (1) présente au moins un élément ressort (18) qui est relié fonctionnellement au corps de seringue (3) et s'oppose au déplacement relatif du corps de seringue (3) par rapport à l'élément manchon (6).

9. Corps de seringue (3) selon l'une des revendications précédentes,
**caractérisé par le fait que**
la coulisse de guidage (12) comporte une première (19) et une seconde région de coulisse (20) qui sont séparées l'une de l'autre par une ligne de séparation fictive (21) s'étendant le long de la direction axiale (X) du corps de seringue (3), dans lequel la saillie de guidage (11) est apte être disposée dans la première région de coulisse (19) dans une position de départ et est transférable de la première région de coulisse (9) dans la seconde région de coulisse (20) dans une position de fin par dépassement de la ligne de séparation (21), lorsqu'une extrémité distale (22) du moyen piquant (5) est disposée au niveau de l'ouverture distale (23) de l'élément manchon (6) lors du déplacement relatif du corps de seringue (3) par rapport à l'élément manchon (6).

10. Corps de seringue (3) selon la revendication 9,
**caractérisé par le fait que**
ladite au moins une saillie de guidage (11) est transférable de la seconde région de coulisse (20) au moyen d'une coulisse de la seconde région de coulisse (20) dans une région d'extrémité (24), dans laquelle un déplacement relatif de l'élément manchon (6) par rapport au corps de seringue (3) est au moins limité sensiblement le long de la direction axiale (X).
